# EUROPEAN PATENT SPECIFICATION

(11) **EP 1 565 451 B1**
(45) Date of publication and mention of the grant of the patent: **09.05.2007**
(21) Application number: 03811772.7
(22) Date of filing: 25.11.2003
(51) Int. Cl.: C07D 333/28, A61K 31/381, A61P 25/00

(54) **HALOTHIENOYL-CYCLOPROPANE-1-CARBOXYLIC ACID DERIVATIVES**
HALOTHIENOYLCYCLOPROPAN-1-CARBONSAEURE-DERIVATE
DERIVES D'ACIDE HALOTHIENOYL-CYCLOPROPANE-1-CARBOXYLIQUE

(30) Priority: 28.11.2002 EP 02026597
(43) Date of publication of application: 24.08.2005
(73) Proprietor: Newron Pharmaceuticals S.p.A., 20091 Bresso (MI) (IT)
(72) Inventor: BENATTI, Luca, I-20091 Bresso (MI) (IT); FARIELLO, Ruggero, I-20091 Bresso (MI) (IT); SALVATI, Patricia, I-20091 Bresso (MI) (IT); PELLICCIARI, Roberto, I-20091 Bresso (MI) (IT); CACCIA, Carla, I-20091 Bresso (MI) (IT)
(74) Representative: Minoja, Fabrizio
(86) International application number: PCT/EP2003/013244
(87) International publication number: WO 2004/048361

(56) References cited:
- WO-A-98/18760
- WO-A-98/40344
- U. SCHMIDT ET AL: "Cyclopropan-Verbindungen aus Phosphoenolbrenztraubensäuretriäthylester" JUSTUS LIEBIGS ANNALEN DER CHEMIE., vol. 733, 1970, pages 180-186, XP002239349 VERLAG CHEMIE GMBH. WEINHEIM., DE ISSN: 0075-4617

## Description

The present invention refers to halothenoyl-cyclopropane-1-carboxylic acid derivatives as long lasting inhibitors of kynurenine 3-monooxygenase (KMO), which are potent glutamate (GLU) release inhibitors.

### BACKGROUND OF THE INVENTION

Metabolites of the kynurenine pathway of tryptophan degradation have been suggested to play an important role in the pathogenesis of several human brain diseases. One of the key metabolites in this pathway, kynurenine, (KYN), is either transaminated to form kynurenate (KYNA), or hydroxylated to the free radical generator 3-OH-KYN. The latter is further degraded to the excitotoxic NMDA receptor agonist QUIN (3-hydroxyanthranilate oxygenase). 3-OH-KYN and QUIN act synergistically, i.e. 3-OH-KYN significantly potentiates the excitotoxic actions of QUIN. The key enzymes in the mammalian brain responsible for the biosynthesis of 3-OH-KYN, (kynurenine 3-monooxygenase, KMO; E.C.1.14.13.9), QUIN and KYNA (kynurenine aminotransferases (KATs I and II) have been characterized and cloned. KMO is a flavin-containing enzyme localized in outer mitochondria membranes of the liver, placenta, spleen, kidney and brain.

Studies from several laboratories have provided evidence that the shift of KYN pathway metabolism away from the 3-OH-KYN/QUIN branch to increase the formation of the neuroprotectant KYNA in the brain leads to neuroprotection.

Elevations in the brain content of KYNA are of particular interest, since they define KMO as a new molecular target for drug development in the area of neuroprotection. The working mechanism is that inhibition of KMO blocks the synthesis of neurotoxins 3-OH-KYN and QUIN, causes accumulation of KYN upstream the metabolic block, and redirect the metabolism of this latter towards the neuroprotectant KYNA.

Notably, it has been reported that KMO expression increases in inflammatory conditions or after immune stimulation (Saito et al. 1993, *J. Biol. Chem.* 268, 15496.-15503; Chiarugi et al 2001, *Neuroscience* 102; 687-695). 3-OH-KYN, the product of its activity, accumulates in the brain of vitamin B-6 deficient neonatal rats (Guilarte and Wagner, 1987, *J. Neurochem.* 49, 1918-1926) and it causes cytotoxicity when added to neuronal cells in primary cultures (Eastman and Guilarte, 1989, *Brain Res.* 495, 225.-231) or when locally injected into the brain (Nakagami et al. 1996, *Jpn. J. Pharmacol.* 71, 183.-186). Recently, it was reported that relatively low concentrations (nanomolar) of 3-OH-KYN may cause apoptotic cell death of neurons in primary neuronal cultures. Structure-activity studies have in fact shown that 3-OH-KYN, and other *o*-amino phenols, may be subject to oxidative reactions initiated by their conversion to quinoneimines, a process associated with concomitant production of oxygen-derived free radicals (Hiraku et al. 1995 *Carcinogenesis* 16, 349-356). The involvement of these reactive species in the pathogenesis of ischemic neuronal death has been widely studied in the last several years and it has been shown that oxygen derived free radicals and glutamate mediated neurotransmission co-operate in the development of ischemic neuronal death (Pellegrini-Giampietro et al. 1990, *J. Neurosci.* 10, 1035-1041).

It was also recently demonstrated that KMO activity is particularly elevated in the iris-ciliary body and that neo-formed 3-OH-KYN is secreted into the fluid of the lens. An excessive accumulation of 3-OH-KYN in the lens may cause cataracts and KMO inhibitors may prevent this accumulation (Chiarugi et al. 1999; *FEBS Letters,* 453; 197-200).

As already mentioned, KMO activity is required for tryptophan catabolism and synthesis of quinolinic acid (QUIN). QUIN is an agonist of a subgroup of NMDA receptors (Stone and Perkins, 1981 *Eur. J. Pharmacol.* 72, 411-412) and when directly injected into brain areas it destroys most neuronal cell bodies sparing fibers *en passant* and neuronal terminals (Schwarcz et al. 1983 *Science* 219, 316-318). QUIN is a relatively poor agonist of the NMDA receptor complex containing either NR2C or NR2D subunits, while it interacts with relatively high affinity with the NMDA receptor complex containing NR2B subunits (Brown et al. 1998, *J. Neurochem.* 71, 1464-1470). The neurotoxicity profile found after intrastriatal injection of QUIN closely resembles that found in the basal nuclei of Huntington's disease patients: while most of the intrinsic striatal neurons are destroyed, NADH-diaphorase-staining neurons (which are now considered able to express nitric oxide synthetase) and neurons containing neuropeptide Y seem to be spared together with axon terminals and fiber *en passant* (Beal et al. 1986 *Nature* 321, 168-171).

*In vitro,* the neurotoxic effects of the compound have been studied in different model systems with variable results: chronic exposure of organotypic cortico-striatal cultures to submicromolar concentration of QUIN causes histological signs of pathology (Whetsell and Schwarcz, 1989, *Neurosci. Lett.* 97, 271-275), similar results have been obtained after chronic exposure of cultured neuronal cells (Chiarugi et al 2001, *J. Neurochem.* 77, 1310-1318).

In models of inflammatory neurological disorders such as experimental allergic encephalitis (Flanagan et al. 1995, *J. Neurochem.* 64, 1192-1196), bacterial and viral infections (Heyes et al. 1992 *Brain* 115, 1249-1273; Espey et al. 1996, *AIDS* 10, 151-158), forebrain global ischemia or spinal trauma, brain QUIN levels are extremely elevated (Heyes and Nowak, 1990 *J. Cereb. Blood Flow Metab.* 10, 660-667; Blight et al. 1995 *Brain* 118, 735-752). This increased brain QUIN concentration could be due to either an elevated circulating concentration of the excitotoxin or to an increased de novo synthesis in activated microglia or in infiltrating macrophages. In retrovirus-infected macaques, it has been proposed that most of the increased content of brain QUIN (approximately 98%) is due to local production. In fact, a robust increase in the activities of IDO, KMO and kynureninase has been found in areas of brain inflammation (Heyes et al. 1998; *FASEB J.* 12, 881-896).

Previous studies have shown that agents able to increase brain KYNA content cause sedation, mild analgesia, increase in the convulsive threshold and neuroprotection against excitotoxic or ischemic damage (Carpenedo et al 1994 *Neuroscience* 61, 237-244; Moroni et al. 1999 *Eur. J. PharniacoL* 375, 87-100; Cozzi et al. 1999; *J, Cereb. Blood Flow & Metab.* 19, 771-777).

In addition to the above reported evidences, it has been recently demonstrated that a number of compounds able to increase brain KYNA formation may cause a robust decrease in glutamate (GLU) mediated neurotransmission by reducing GLU concentrations in brain extracellular spaces (Carpenedo et al 2001, *Eur. J. Neuroscience* 13, 2141-2147).

Compounds endowed with KMO inhibiting activity may therefore be used for the treatment of a number of degenerative or inflammatory conditions in which an increased synthesis in the brain of QUIN, 3-OH-KYN are involved and may cause neuronal cell damage. These compounds in fact prevent the synthesis of both 3-OH-KYN and QUIN by inhibiting the KMO enzyme, and concomitantly cause KYNA to increase in the brain.

2-substituted benzoyl-cycloalkyl-1-carboxylic acid derivatives having KMO inhibiting activity are disclosed in WO 98/40344. In particular one of said compounds, 2-(3,4-dichlorobenzoyl)-cyclopropane-1-carboxylic acid, was reported to have an interesting activity, with an IC₅₀ for KMO inhibition of 0.18 µM, but its potency and pharmacokinetic properties were less than satisfactory.

### DESCRIPTION OF THE INVENTION

It has now been found that some derivatives of halothenoyl-cyclopropane-1-carboxylic acids have favourable and long lasting activities on both KMO and GLU release.

The present invention accordingly provides compounds of formula (**I**) 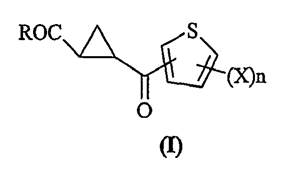 wherein
R is hydroxy, linear or branched C₁-C₆ alkoxy, phenoxy, benzyloxy, a group -N(R¹R²) wherein R¹ is hydrogen, linear or branched C₁-C₄ alkyl, benzyl, phenyl and R² is hydrogen or linear or branched C₁-C₄ alkyl, or R is a glycoside residue or a primary alkoxy residue from ascorbic acid, optionally having one or more hydroxy groups alkylated or acylated by linear or branched C₁-C₄ alkyl or acyl groups;
X is a halogen atom selected from the group consisting of fluorine, chorine or bromine, preferably chlorine;
n is an integer of 1 or 2
   and pharmaceutically acceptable salts thereof.

The term "glycoside residue" means a mono-, di- or oligosaccharide.

Among compounds of formula (**I**) wherein R is a glycoside residue, R is preferably an optionally alkylated or acylated beta D-glucopyranosyloxy or 6-deoxygalactopyranosyloxy residue. The galactopyranosyl residue is particularly preferred.

Preferred compounds of formula (**I**) are those wherein R is hydroxy, methoxy or ethoxy and X is chlorine. Particularly preferred are compounds of formula (**I**) selected from:
2-(2-chloro-4-thenoyl)-cyclopropane-1-carboxylic acid,
methyl-2-(2-chloro-4-thenoyl)-cyclopropane-1-carboxylate,
ethyl-2-(2-chloro-4-thenoyl)-cyclopropane-1-carboxylate,
2-(3-chloro-4-thenoyl)-cyclopropane-1-carboxylic acid,
methyl-2-(3-chloro-4-thenoyl)-cyclopropane-1-carboxylate,
ethyl-2-(3 -chloro-4-thenoyl)-cyclopropane-1 -carboxylate,
2-(2-chloro-5-thenoyl)-cyclopropane-1-carboxylic acid,
methyl-2-(2-chloro-5-thenoyl)-cyclopropane-1-carboxylate,
ethyl-2-(2-chloro-5-thenoyl)-cyclopropane-1-carboxylate,
2-(3-chloro-5-thenoyl)-cyclopropane-1-carboxylic acid,
methyl-2-(3-chloro-5-thenoyl)-cyclopropane-1-carboxylate,
ethyl-2-(3-chloro-5-thenoyl)-cyclopropane-1-carboxylate,
2-(2,3-dichloro-4-thenoyl)-cyclopropane-1-carboxylic acid,
methyl-2-(2,3-dichloro-4-thenoyl)-cyclopropane-1-carboxylate,
ethyl-2-(2,3-dichloro-4-thenoyl)-cyclopropane-1-carboxylate.
2-(2,3-dichloro-5-thenoyl)-cyclopropane-1-carboxylic acid,
methyl-2-(2,3-dichloro-5-thenoyl)-cyclopropane-1-carboxylate,
ethyl-2-(2,3-dichloro-5-thenoyl)-cyclopropane-1-carboxylate

Pharmaceutically acceptable salts of compounds of formula (**I**) wherein R is hydroxy include salts with inorganic bases, e.g. alkali metal bases, especially sodium or potassium bases or alkaline-earth metal bases, especially calcium or magnesium bases, or with pharmaceutically acceptable organic bases.

The present invention includes within its scope all the pure possible isomers of compounds of formula (**I**) and the mixtures thereof. Particularly preferred are *trans* isomers, more preferred S,S-isomers.

The invention also concerns pharmaceutical compositions comprising a compound of formula (**I**) as the active ingredient as well as the use of compounds (**I**) for the preparation of medicaments for use as kynurenine-3-hydroxylase inhibitors.

Compounds of formula (**I**) wherein R is hydroxy, methoxy or ethoxy can be obtained by a process comprising the following steps and illustrated in Scheme 1:
a) monohydrolysis of dimethyl- or diethyl cyclopropane carboxylate (**II**) to give methyl- or ethyl cyclopropane carboxylate (**III**);
b) conversion of methyl- or ethyl cyclopropane carboxylate into a compound of formula (**IV**) by treatment with N-methyl-N-methoxamine hydrochloride;
c) treatment of compound (**IV**) with a suitable Grignard compound of formula (**V**) wherein X and n have the meanings above defined and X' is bromine or iodine to give a compound of formula (**I**) wherein R is methoxy or ethoxy;
d) basic hydrolysis of compound (**I**) to give a compound of formula (**I**) wherein R is hydroxy.

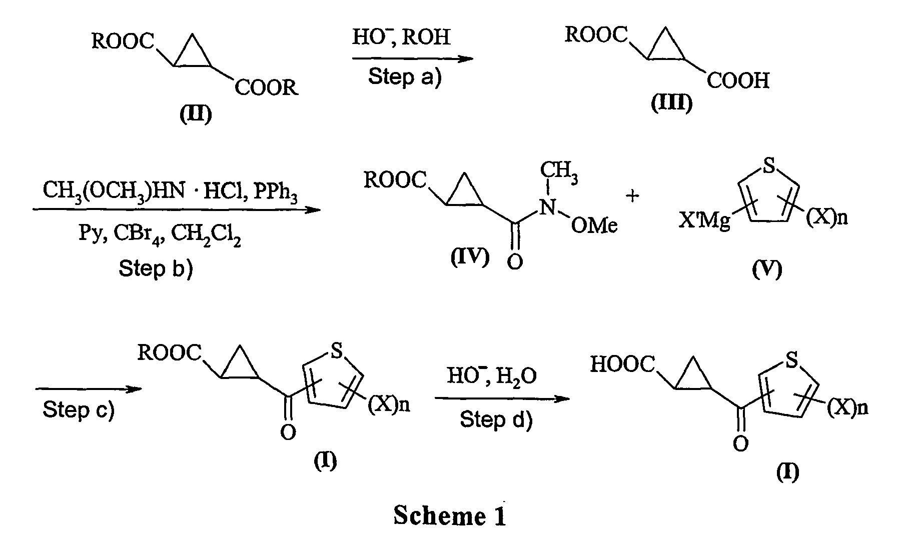

Step a) is carried out by treating compound (**II**) with NaOH or KOH, preferably KOH, in methanol or ethanol under reflux. Compound (**III)** can be used for the following step without any further purification.
Step b) is carried out by reacting compound (**III**) in N-methyl-N-methoxyamine hydrochloride, CBr₄, pyridine, PPh₃ and methylene chloride at room temperature. The reaction affords compounds (**IV**) in 55-75% yield.
Step c) can be carried out in any solvent suitable for Grignard's reactions, preferably in THF at room temperature. More specifically, for the preparation of methyl- or ethyl-2-(2-chloro-4-thenoyl)-cyclopropane-1-carboxylate, step c) is carried out by reacting compound (**IV**) with 4-bromo-2-chloro-thiophene and magnesium powder in THF. 4-Bromo-2-chloro-thiophene can be prepared either according to the procedure described in Dettmeier et al, Angew Chem, Int. Ed. Engl. 1987, 26, 548 or by a process (Scheme 2) comprising the reaction of 2,3-dibromothiophene with N-chlorosuccinimide in an acidic medium, preferably acetic acid, under reflux to afford 2,3-dibromo-5-chloro-thiophene, which is treated with butyllitium and hydrolysed.

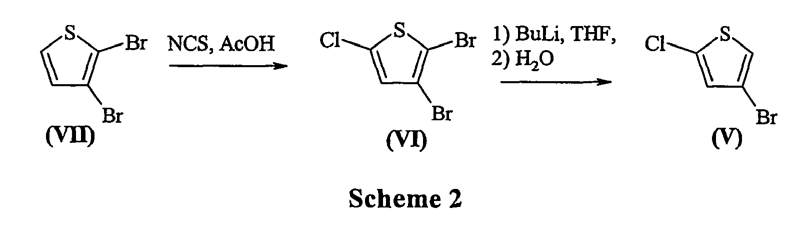

This synthetic methodology represents a highly efficient and cheap route to prepare ketones from carboxylic acids and can be applied also with optically active compounds, because the formation of compound (**IV**) doesn't cause racemization. This allows to obtain enantiomerically pure compounds of formula (**I**) when starting from enantiomerically pure dimethyl or diethyl cyclopropane carboxylate, which can be obtained with conventional methods from succinic anhydride and *l*- or *d*-menthol, as hereinafter described in more detail in the examples.

Step d) is carried out with any conventional method suitable for esters hydrolysis. According to a preferred embodiment of the invention, the hydrolysis is carried out in aqueous potassium hydroxyde in dioxane.

Compounds of formula (**I**) wherein R is other than hydroxy, methoxy or ethoxy can be obtained from compounds of formula (**I**) wherein R is hydroxy, methoxy or ethoxy by conventional methods of preparation of esters or amides.

Compounds of formula (**I**) wherein R is a glycoside or an ascorbic acid residue can be prepared by a process comprising the reaction of a compound of formula (**I**) in which R is hydroxy with suitably protected saccharide or ascorbic acid derivatives, optionally followed by the removal of the protective groups present on the saccharide or ascorbic acid hydroxy groups.

Examples of suitable saccharide derivatives include 1,2,3,4-di-O-isopropylidene-galactopyranose, 1,2,3,4-di-O-isopropylidene-glucopyranose, glucopyranosyl bromide tetraacetate or tetrabenzoate, glucopyranosyl chloride tetraacetate or tetrabenzoate, galactopyranosyl bromide tetraacetate or tetrabenzoate, galactopyranosyl chloride tetraacetate or tetrabenzoate and the like. Preferably, compounds (**I**) in which R is hydroxy is reacted with 1,2,3,4-di-O-isopropylidene-galactopyranose or glucopyranose in the presence of a condensing agent such as carbonyldiimidazole, dicyclohexylcarbodiimide or the like, in anhydrous solvents and under inert atmosphere. The obtained compounds may then be transformed into the desired compounds of formula (**I**) by treatment with organic acids, e.g. with trifluoroacetic or trichloroacetic acid in halogenated hydrocarbons, ethers, aliphatic or aromatic hydrocarbons, etc.

Pharmaceutically acceptable salts of compounds of formula (**I**) wherein R is hydroxy can be obtained by conventional methods using an inorganic or an organic base.

Compounds of formula (**I**) are potent KMO inhibitors and can modify the formation of all the neuroactive compounds formed along the pathway. In particular, they inhibit the formation of 3-OH-KYN and its metabolites in the pathway leading to QUIN. More particularly, the compounds of this invention are able to increase brain KYNA content and to decrease excitatory glutamatergic neurotransmission with a long lasting and particularly favourable time course.

The compounds of the invention may therefore be used for the treatment of a number of degenerative or inflammatory conditions in which an increased synthesis in the brain of QUIN, 3-OH-KYN or increased release of GLU are involved and may cause neuronal damage. Examples of said conditions include:
neurodegenerative disorders including Parkinson's syndrome, Huntington's chorea, Senile Dementia Alzheimer's type, Amiotrophic Lateral Sclerosis;
inflammatory disorders of the central and/or peripheral nervous system including multiple sclerosis (see: Chiarugi et al. *Neuroscience* 2001, 102, 687-695; Chiarugi et al. *J*. *Leukoc. Biol.* 2000, 68, 260-266), Guillain Barrè Syndrome and other neurophaties;
infectious disease caused by viral (including AIDS see: Heyes et al. *Annals Neurol.* 1991, 29, 202-209), bacteria and other parasites including malaria, septic shock, etc.;
immunitary disorders and therapeutic treatment aimed at modifying biological responses (for instance administrations of interferons or interleukins, see: Brown et al. *Cancer Res.* 1989, 49, 4941-4945);
neoplastic disorders including lymphomas and other malignant blood disorders;
convulsive Disorders, including variants of *Grand mal* and *petit mal* epilepsy and Partial Complex epilepsy (see: Carpenedo et al. 1994, *Neuroscience* 61, 237-244);
ischemic disorders including stroke (focal ischemia);
cardiac arrest or insufficiency and hemorrhagic shock (global brain ischemia), carbon monoxide poisoning, near drawning (see: Cozzi et al. 1999, *J. Cereb. Blood Flow Metab.* 19, 771-777);
traumatic damage to the brain and spinal cord;
tremor syndromes and different movement disorders (diskynesia);
psychiatric disorders including anxiety, insomnia, depression and schizophrenia;
nicotine addiction (kynurenate is an antagonist of nicotinic receptors). Other addictive disorders including alcoholism, cannabis, benzodiazepine, barbiturate, morphine and cocaine dependence (see: Albuquerque et al. 2001, *J. Neurosci.* 21, 7463-7473);
cataract formation and aging of the eye (see: Chiarugi et al. 1999; *FEBS Letters* 453, 197-200).

For the considered therapeutic uses, the compounds of the invention will be administered to the affected patients in form of pharmaceutical compositions suitable for the oral, parenteral, transmucosal or topical administration.

The pharmaceutical compositions may be prepared following conventional methods.

For example, the solid oral forms may contain, together with the active compound, diluents, e.g. lactose, dextrose, saccharose, sucrose, cellulose, corn starch or potato starch; lubricants, e.g. silica, talc, stearic acid, magnesium or calcium stearate, and/or polyethylene glycols; binding agents, e.g. starches, arabic gum, gelatin, methyl cellulose, carboxymethyl cellulose or polyvinyl pyrrolidone; disaggregating agents, e.g. a starch, alginic acid, alginates or sodium starch glycolate; effervescing mixtures; dyestuffs; sweeteners; wetting agents such as lecithin, polysorbates, lauryl sulfates; and, in general, non-toxic and pharmacologically inactive substances used in pharmaceutical formulations. Said pharmaceutical preparations may be manufactured in known manner, for example, by means of mixing, granulating, tabletting, sugar-coating, or film-coating processes.

The liquid dispersions for oral administration may be e.g. syrups, emulsions and suspensions.

The syrups may contain as carrier, for example, saccharose or saccharose with glycerine and/or mannitol and/or sorbitol.

The suspensions and the emulsions may contain as carrier, for example, a natural gum, agar, sodium alginate, pectin, methylcellulose, carboxymethyl cellulose, or polyvinyl alcohol.

The suspension or solutions for intramuscular injections may contain a pharmaceutically acceptable carrier, e.g. sterile water, olive oil, ethyl oleate, glycols and optionally local anaesthetics. The solutions for intravenous injections or infusions may contain as carrier, for example, sterile water, isotonic saline solutions or propylene glycol.

The suppositories may contain a pharmaceutically acceptable carrier, e.g. cocoa butter, polyethylene glycol, a polyoxyethylene sorbitan fatty acid ester surfactant or lecithin.

For the use in ophthalmology, the compounds may be formulated as eye-drops in a sterile carrier, usually an isotonic saline solution.

The dosage level will depend on the age, weight, conditions of the patient and on the administration route even though it will typically range from about 10 to about 1000 mg pro dose, from 1 to 5 times daily.

The following examples illustrate the invention in more detail.

### EXAMPLES

### Material and methods

Melting points were determined on a Buchi 535 hot-stage apparatus and are uncorrected. ¹H-NMR and ¹³C-NMR spectra were performed on a Brucker AC 200 spectrometer, the chemical shifts are in ppm downfield from tetramethylsilane. Flash chromatography was performed on Merck silica gel (0.040-0.063 mm). Toluene was distilled from sodium; tetrahydrofuran was distilled from sodium/benzofenone and then from lithium aluminium hydride; methanol was distilled from magnesium; methylene chloride was distilled from lithium aluminium hydride. Oxalyl chloride and 2,2,6,6-tetramethylpiperidine were distilled before use. Isobutyraldehyde, bromochloromethane, o-dichlorobenzene, 2,3-dibromothiophene and 2-chloro-5-bromothiophene were purchased best grade from Aldrich and used without purification.

### Example 1

### (±)-trans-Cyclopropane-1,2-dicarboxylic acid monomethyl ester

A methanolic solution of potassium hydroxide (814 mg, 14.5 mmol) was added to a solution of 2.09 g (13.2 mmol) of dimethyl (±)-*trans-*cyclopropane-1,2-dicarboxylate in methanol. The mixture was refluxed for 5 h, allowed to cool at room temperature, poured into water and extracted with ethyl acetate. The inorganic layer was acidified to pH 2 with 10% HCl and extracted again with ethyl acetate. The combined organic layers were washed with a saturated sodium chloride solution, dried over anhydrous sodium sulfate and concentrated with a rotary evaporator. The crude product (1.32 g, yield 69%) was used for the following step.
¹H-NMR (200 MHz; CDCl₃) δ (ppm): 1.45 (m, 2 H, *CH₂);* 2.30-2.39 (m, H, *CH);* 3.32-3.41 (s, H, *CH);* 3.69 (s, 3 H, *CH*₃).

### Example 2

### (±)-trans-Cyclopropane-1,2-dicarboxylic acid monoethylester

To a solution of diethyl (+)-trans-cyclopropane- 1 ,2-dicarboxylate, (1.60 g, 8.53 mmol) in ethanol (10 ml) a solution of potassium hydroxide (503 mg, 8.96 mmol) in ethanol (5 ml) was added in one portion and the reaction mixture was refluxed for 5 h. Then, after cooling to room temperature, the reaction mixture was poured into water and extracted three times with ethyl acetate. The aqueous layer was acidified with 10% HCl, then extracted with diethyl ether. The combined organic extracts were washed with a saturated solution of sodium chloride and dried over anhydrous sodium sulfate. The solvent was eliminated with a rotary evaporator. The crude product (1.10 g, 82% yield) was used in the following reaction without any further purification.
¹H-NMR (200 MHz; CDCl₃) δ (ppm): 1.17-1.24 (t, 3 H, J = 7.1 Hz, OCH₂*CH₃*); 1.40-1.47 (m, 2 H, *CH₂);* 2.09-2.19 (m, 2 H, *CH, CH*); 4.08-4.17 (q, 2H, J = 7.1 Hz, O*CH*₂CH₃).

### Example 3

### Methyl-(±)-trans-[2-(N-methoxy-N-methyl)-aminocarbonyl]cyclopropane-1-carboxylate

N-methoxy-N-methylamine hydrochloride (0,955, 9.84 mmol), pyridine (0.88 ml, 9.84 mmol), carbon tetrabromide (3.27 g, 9.84 mmol) and triphenylphosphine were added subsequently and in portions to a solution of (±)-*trans*-cyclopropane-1,2-dicarboxylic acid monomethyl ester (1.29 g, 8.95 mmol) in 25 ml of dichloromethane.

The mixture was stirred under argon atmosphere at room temperature for 14 h, then the solvent was evaporated off. The residue was taken up with diethyl ether and the precipitated phosphinoxide was filtered off, then the filtrate was concentrated under vacuum. The crude residue was purified by flash chromatography on silica gel (eluant petroleum ether/ethyl acetate 7/3) affording 1.03 g of the title compound (yield 61%).
¹H-NMR (200 MHz; CDCl₃) δ (ppm): 1.32-1.45 (m, 2 H, CH₂); 2.09-2.19 (m, H, *CH);* 2.65 (bm, H, *CH),* 3.17 (s, 3H, *CH₃N);* 3.67 (s, 3 H, *CH₃O),* 3.71 (s, 3 H, *CH₃O).*
¹³C-NMR (200 MHz; CDCl₃), δ (ppm): 15.18, 19.79, 21.73, 32.55, 52.08, 61.77, 171.248, 173.294.

### Example 4

### Ethyl (±)-trans-[2-(N-methoxy-N-methyl)-aminocarbonyl]-cyclopropane-1-carboxylate

To a solution of (±)-*trans*-cyclopropane-1,2-dicarboxylic acid monoethylester (1.10 g, 6.98 mmol), in 20 ml of methylene chloride N-methoxy-N-methylamine hydrochloride (0.749 g, 7.68 mmol), pyridine (620 µl, 7.68 mmol), carbon tetrabromide (2.547 g, 7.68 mmol) were added then triphenylphosphine (2.014 g, 7.68 mmol) portionwise. The reaction mixture was strirred for 14 h under argon atmosphere at room temperature then concentrated under vacuum. The residue was taken up with diethyl ether and the solid precipitated was filtered. The filtrate was concentrated under vacuum. The crude product was purified by flash chromatography on silica gel (petroleum ether/ethyl acetate = 7/3) thus affording 3.086 g of the title compound (73% yield).
¹H-NMR (200 MHz; CDCl₃) δ (ppm): 1.16-1.24 (t, 3 H, J = 7.1 Hz, OCH₂*CH*₃); 1.30-1.41 (m, 2 H, *CH₂*); 2.05-2.14 (m, H, *CH);* 2.60 (bm, H, *CH*), 3.14 (s, 3H, *CH₃N*); 3.68 (s, 3 H, *CH₃O*), 4.03-4.14 (s, 2 H, J = 7.1 Hz, O*CH*₂CH₃).

### Example 5

### Methyl (±)-trans-2-(2-chloro-4-thenoyl)-cyclopropane-1-carboxylate

A 2 M solution of a Grignard compound freshly prepared from 2-chloro-4-bromothiophene in THF (2.5 ml) was added to a solution of methyl-(±)-*trans*-[2-(N-methoxy-N-methyl)-aminocarbonyl]cyclopropane-1-carboxylate (250 mg, 1.34 mmol) in THF (1,5 ml) at 0°C. The mixture was stirred under argon at room temperature for 14 hours, then a solution (4 ml) of ethanol: 10% HCl 1:1 was added. The aqueous layer was extracted with ethyl acetate and the combined organic layers were washed with a sodium chloride saturated solution and dried over anhydrous sodium sulfate.

The solvent was evaporated off and the residue was purified by flash chromatography on silica gel (eluant: petroleum ether/ethyl acetate 95/5) affording 145 mg of compound the title compound (yield 44%).
¹H-NMR (200 MHz; CDCl₃) δ (ppm): 1.48-1.62 (m, 2 H, *CH₂);* 2.28-2.38 (m, H, *CH);* 2.86-2.95 (m, H, *CH);* 3.71 (m, 3 H, *CH₃O),* 7.37-7.38 (d, H, J = 2 Hz, *CH*), 7.91-7.92 (d, H, J = 2 Hz, *CH*).
¹³C-NMR (200 MHz; CDCl₃) δ (ppm): 17.71; 17.71-24.30 (J = 1318 Hz); 26.48; 52.27; 125.56; 130.92; 131.75; 141.28; 172.60; 189.94.

### Example 6

### Ethyl (±)-trans-[2-(2-chloro-4-thenoyl)]-cyclopropane-1-carboxylate

To a solution of ethyl (±)*-trans-*[2*-*(*N*-methoxy-*N*-methyl)-aminocarbonyl]-cyclopropane-1-carboxylate (300 mg, 1.49 mmol) in THF (8 ml) at 0°C a 2.0 M THF solution (2.1 ml) of a Grignard reagent freshly prepared from 2-chloro-4-bromothiophene was added. The reaction mixture was stirred for 2 h under argon atmosphere at 0°C. Then, 8 ml of a 1/1 ethanol/10% HCl solution was added. The two phases were separated and the aqueous layer was extracted three times with ethyl acetate. The combined organic extracts were washed with a saturated solution of sodium chloride and dried over anhydrous sodium sulfate. The solvent evaporated off with a rotary evaporator. The reaction was repeated twice using the same amounts of the reagents. The collected crude products were purified by flash chromatography on silica gel (petroleum ether/ethyl acetate = 95/5) thus affording 0.565 g of the title compound (49% yield).

¹H-NMR (200 MHz; CDCl₃) δ (ppm): 1.23-1.30 (t, 3 H, J = 7.1 Hz, OCH₂*CH*₃); 1.50-1.60 (m, 2 H, *CH₂);* 2.27-2.33 (m, H, *CH*); 2.85-2.95 (m, H, *CH);* 4.10-4.21 (q, 2 H, J = 7.1 Hz, OC*H₂*CH₃), 7.37-7.38 (d, H, J = 2 Hz, *CH),* 7.91-7.92 (d, H, J = 2 Hz, *CH).*

### Example 7

### (±)-trans-2-(2-Chloro-4-thenoyl)-cyclopropan-1-carboxylic acid

An aqueous solution of potassium hydroxide (15 mg, 0.27 mmol) was added to a solution of methyl (±)-*tran*s*-*2-(2-chloro-4-thenoyl)-cyclopropane-1-carboxylate (65 mg, 0.27 mmol) in dioxane and the mixture was stirred at room temperature for 5 h. After addition of water (1 ml) the mixture was extracted with ethyl acetate. The combined organic layers were washed with a saturated solution of sodium chloride, dried over anhydrous sodium sulfate and the solvent was evaporated off. The product was minced with n-hexane, filtered under reduced pressure and dried with a high vacuum pump, affording 37 mg of pure title compound (yield 60%). p.f. = 136-138°C
¹H-NMR (200 MHz; CDCl₃+CD₃OD) δ (ppm): 1.56-1.69 (m, 2 H); 2.29-2.38 (m, H); 2.92-3.01 (m, H); 7.38-7.39 (d, H, J = 2 Hz), 7.93-7.94 (dd, H, J = 2 Hz).
¹³C-NMR (400 MHz; CDCl₃+CD₃OD) δ (ppm): 17.98, 23.89, 26.88, 125.55, 131.06, 131.92, 141.12, 177.37, 189.98.

| | |
|---|---|
| Elem. anal.: | (calculated) C %: 47.25; H %: 3.06; |
| | (found) C %: 46.80; H %: 3.24. |

### Example 8

### (±)-trans-2-(2-Chloro-4-thenoyl)-cyclopropan-1-carboxylic acid

To a solution of ethyl (±)-*trans*-2-(2-chloro-4-thenoyl)-cyclopropane-1-carboxylate (0.551 g, 2.13 mmol) in dioxane (15 ml) a solution of potassium hydroxide (1.175 g, 3.12 mmol) in water (7 ml) was added. The reaction mixture was stirred at room temperatur for 5 h. Then, 5 ml of water added, the two phases were separated and the aqueous layer was extracted once with ethyl acetate. The aqueous layer was acidified with 10% HCl, then extracted three times with diethyl ether. The combined organic extracts were washed with a saturated solution of sodium chloride and dried over anhydrous sodium sulfate. The solvent was eliminated with a rotary evaporator. The crude product was minced with n-hexane, filtered under reduced pressure and dried with a high vacuum pump. 0.426 g of pure title product was obtained (87% yield).
mp = 136-138°C
¹H-NMR (200 MHz; CDCl₃+CD₃OD) δ (ppm): 1.56-1.69 (m, 2 H); 2.29-2.38 (m, H); 2.92-3.01 (m, H); 7.38-7.39 (d, H, J = 2 Hz), 7.93-7.94 (dd, H, J = 2 Hz).
¹³C-NMR (400 MHz; CDCl₃+CD₃OD) δ (ppm): 17.98, 23.89, 26.88, 125.55, 131.06, 131.92, 141.12, 177.37, 189.98.

| | |
|---|---|
| Elem. Anal.: | (theor.) C %: 47.25; H %: 3.06; |
| | (exper.) C %: 47.00; H %: 3.15. |

### Example 9

### 2,3-dibromo-5-chlorothiophene

To a solution of 2,3-dibromothiophene (25g, 103 mmol) in acetic acid (100 ml) N-chlorosuccinimide (14.5 g, 109 mmol) was added in portions (a small aliquot at room temperature and the following under reflux). The mixture was refluxed for 3 h, then allowed to cool to room temperature and poured into water. The aqueous layer was extracted with ethyl ether and the combined organic layers were washed to neutrality with NaOH 2 N, then with a saturated sodium chloride solution and dried over anhydrous sodium sulfate. The solvent was evaporated off under vacuum and the residue, which contained about 52% of 2,3-dibromo-5-chlorothiophene was distilled under vacuum (10 mmHg). The fraction containing 60% of the title product (t = 75-85°C) was used as such for the following step.
¹H-NMR (200 MHz; CDCl₃) δ (ppm): 6.76 (s, H, *CH).*

### Example 10

### 4-bromo-2-chloro-thiophene

Butyllithium 2.4 M in hexane (8.5 ml) was added to a solution of 2,3-dibromo-5-chlorothiophene (7.21 g) in THF (20 ml) at -78°C. After 10' from the end of the addition, the mixture was allowed to stand at room temperature and 10 ml of water were added. The aqueous layer was extracted with ethyl ether, then the combined organic layers were washed with a saturated solution of sodium chloride, dried over anhydrous sodium sulfate and the solvent was distilled off under vacuum at room temperature. The residue was distilled under vacuum and the fractions enriched in the title compound were combined (2.26 g) and used as such.

¹H-NMR (200 MHz; CDCl₃) δ (ppm): 6.83-6.84 (d, H, J = 2 Hz, *CH*), 7.00-7.01 (d, H, J = 2 Hz, *CH).*

### Example 11

### (-)-Dimenthyl Succinate

To a solution of succinic anhydride (15.2 g, 0.152 mol) and *l*-menthol (47.5 g, 0.304 mol) in dry toluene (120 ml), under magnetic stirring, p-toluensulfonic acid (0.190 g, 1.04 10⁻³ mol) was added. The mixture was refluxed for 24 h and the theoretical amount of water (2.73 ml) was collected. The cooled mixture was diluited with petroleum ether (200 ml) and poured into a 2.5:1:2 mixture of saturated aqueous sodium bicarbonate solution, methanol and water (550 ml). The organic phase was separated and the aqueous layer extracted with petroleum ether (3x100 ml). The collected organic layers were washed with saturated sodium chloride (1x100 ml) and dried over sodium sulphate. The solvent was distilled off with a rotatory evaporator and the resulting crude product recrystallized from methanol. 54 g of pure (-)-dimenthyl succinate were obtained (89%).
mp: 61-62°C
[α]_{D}²⁵ = -87 (c = 1, CHCl₃)

### Example 12

### (+)-Dimenthyl (1S, 2S)-Cyclopropane-1,2-dicarboxylate.

A 2.5 M solution of butyllithium in hexane (56.9 ml, 142.2 mmol) was added to 180 ml of dry tetrahydrofuran (THF), cooled to -20°C. 2,2,6,6-Tetramethylpiperidine (24 ml, 142.2 mmol) was added dropwise over a period of 10 minutes. The resulting solution of lithium 2,2,6,6-tetramethylpiperidide (LTMP) was cooled to -78°C and stirred for 30 minutes. A solution of (-)-dimenthyl succinate (26.75 g, 67.7 mmol) in THF (60 ml) was then added over a period of 1 h. The resulting yellow solution was stirred for 1 h. Thereafter, bromochloromethane (4.39 ml, 67.7 mmol) was added and the reaction mixture stirred for 2 h. The reaction was quenched by adding isobutyraldehyde (22.46 ml, 27.08 mmol). After stirring for further 30 minutes, the mixture was poured into ice-cooled 1N hydrochloric acid (250 ml) and the aqueous layer was extracted with diethyl ether (3x150 ml). The combined organic layers were washed with saturated sodium chloride (250 ml), dried over sodium sulphate and concentrated with a rotary evaporator. The residue was chromatographed on silica gel (petroleum ether/diethyl ether = 98/2). An additional flash chromatography on silica gel (petroleum ether/diethyl ether = 98/2) afforded the pure title compound.
Yield 33%
mp: 95-96°C
[α]_{D}²⁵ = -18.8 (c = 1, CHCl₃)
¹H-NMR (CDCl₃) δ: 0.70-2.20-(complex, 20 H); 0.75 (d, 6H, J = 7 Hz); 0.9 (d, 9H, J = 6.8 Hz); 2.15 (dd, 2H, J = 7.6, 8.7 Hz); 4.7 (dt, 2H, J = 4.3, 10.7 Hz).
¹³C-NMR (CDCl₃) δ: 15.2; 16.4; 20.6; 21.9; 22.2; 23.6; 26.3; 31.3; 34.2; 40.8; 47.0; 74.9; 171.2.

### Example 13

### (+)-(1S, 2S)-Cyclopropane-1,2-dicarboxylic acid

To a solution of (+)-dimenthyl (1*S*, 2*S*)-cyclopropane-1,2-dicarboxylate (8.8 g, 21.62 mmol) in methanol (38 ml), an aqueous solution (5 ml) of potassium hydroxide (4.32 g; mmol) was added. The mixture was heated to 60°C for 4 h then cooled to room temperature. The reaction mixture was diluited with water (40 ml) and extracted with diethyl ether (4x40 ml). The aqueous layer was acidified with 3 N hydrochloric acid, saturated with sodium chloride and extracted with diethyl ether (6x40 ml). The combined organic layers were dried over sodium sulphate and concentrated with a rotary evaporator. 2.27 g of the title compound were obtained after sublimation (80% yield).
mp: 168-169°C
[α]_{D}²⁰ = +224.9 (c = 1, EtOH)
¹H-NMR (CDCl₃+CD₃OD) δ: 1.45 (t, 2H, J = 8.2 Hz); 2.1 (t, 2H, J = 7 Hz); 7.9 (br, 2H).

### Example 14

### (+)-Dimethyl (1S, 2S)-cyclopropane-1,2-dicarboxylate

A solution of (+)-(1*S*, 2S)-cyclopropane-1,2-dicarboxylic acid (2.2 g, 16.9 mmol) in oxalyl chloride (35 ml) was stirred under argon at room temperature for 4 h.. Oxalyl chloride was then removed with a rotary evaporator and the oily residue dissolved in dry methanol (100 ml). Stirring was continued for 12 h and methanol was evaporated. The residue was chromatographed on silica gel (petroleum ether/ethyl acetate = 85/15-7/3), affording 2.48 g of (+)-dimethyl (1*S*, 2*S*)-cyclopropane-1,2-dicarboxylate (93% yield).
[α]_{D}²⁴ = +218 (c = 5, CH₂Cl₂)
¹H-NMR (CDCl₃) δ: 1.45 (t, 2H, J = 8.2 Hz); 2.1 (t, 2H, J = 7 Hz); 3.65 (s, 6H).
¹³C-NMR (CDCl₃) δ: 15.0; 21.9; 51.8; 171.9.

### Example 15

### (+)-(1S, 2S)-Cyclopropane-1,2-dicarboxylic acid monomethyl ester

A methanolic solution (13 ml) of potassium hydroxide (1.44 g; 25.72 mmol) was added to a solution of (+)-dimethyl (1*S*, 2*S*)-cyclopropane-1,2-dicarboxylate (3.68 g, 23.25 mmol) in methanol (23 ml). After reaction and work-up according to example 1, 2.69 g of the title compound were obtained (80% yield).
[α]_{D}²⁴ = +245 (c = 1, CH₂Cl₂)
¹H-NMR (CDCl₃) δ: 1.45 (m, 2H); 2.15 (m, 2H, J = 7 Hz); 3.65 (s, 3H); 10.7 (br, 1H).

### Example 16

### Methyl (1S, 2S)-trans-[2-(N-methoxy-N-methyl) -aminocarbonyl]-cyclopropane-1-carboxylate

The title compound was prepared following the procedure of example 3, using (+)-(1S, 2S)-cyclopropane-1,2-dicarboxylic acid monomethyl ester as a starting material and carrying out the reaction for 4 hours.
Yield: 69%
¹H-NMR (200 MHz; CDCl₃) δ (ppm): 1.33-1.46 (m, 2 H, *CH₂);* 2.10-2.19 (m, H, *CH*); 2.66 (bm, H, *CH),* 3.18 (s, 3H, *CH₃N);* 3.68 (s, 3 H, *CH₃ O*), 3.72 (s, 3 H, J = 7.1 Hz, *OCH₃).*

### Example 17

### Methyl (1S, 2S)-trans-[2-(2-chloro-4-thenoyl)]-cyclopropane-1-carboxylate

Following the procedure of example 5, the compound of example 16 (0.3 g, 1.60 mmol in 4 ml of THF) was reacted with 2.4 ml of a Grignard reagent freshly prepared from 2-chloro-4-bromothiofene affording 0.124 g of the title compound.
Yield: 32%
¹H-NMR (200 MHz; CDCl₃) δ (ppm): 1.46-1.59 (m, 2 H, *CH₂);* 2.25-2.37 (m, H, *CH);* 2.84-2.93 (m, H, *CH);* 3.68 (s, 3 H, O*CH₃*), 7.33-7.34 (d, H, J = 2 Hz, *CH*), 7.90-7.91 (d, H, J = 2 Hz, *CH).*

### Example 18

### (1S, 2S)-trans-[2-(2-chloro-4-thenoyl)]-cyclopropane-1-carboxylic acid

Following the procedure of example 6, 0.065 g (0,27 mol) of compound of example 17 were reacted with a solution of potassium hydroxide (0.017 g, 0.30 mmol) in water (1 ml), affording 0,049 g of pure title compound.
Yield: 80%
mp = 136-138°C
¹H-NMR (200 MHz; CDCl₃) δ (ppm): 1.54-1.69 (m, 2 H); 2.29-2.38 (m, H); 2.91-3.00 (m, H); 7.37-7.38 (d, H, J = 2 Hz), 7.92-7.93 (d, H, J = 2 Hz).
¹³C-NMR (400 MHz; CDCl₃+CD₃OD) δ (ppm): 17.98, 23.89, 26.88, 125.55, 131.06, 131.92, 141.12, 177.37, 189.98.
e.e. (HPLC λ = 254 nm) >99%

### Example 19

### Methyl (1S, 2S)-trans-[2-(2-Chloro-5-thenoyl)]-cyclopropane-1-carboxylate

To a solution of methyl (*1S,* 2S)-trans-[2-(*N*-methoxy-*N*-methyl)-aminocarbonyl]-cyclopropane-1-carboxylate (0.150 g, 0.80 mmol) in THF (4 ml) at 0°C a 1.0 M diethyl ether solution (0.9 ml) of a Grignard reagent freshly prepared from 2-chloro-4-bromothiofene was added. The reaction mixture was stirred for 1 h under an argon atmosphere at room temperature. Then, 6 ml of a 1/1 methanol/10% HCl solution was added. The two phases were separated and the aqueous layer was extracted three times with diethyl ether. The combined organic extracts were washed with a saturated solution of sodium chloride and dried over anhydrous sodium sulphate. The solvent was eliminated with a rotary evaporator. The crude product was purified by flash chromatography on silica gel (petroleum ether/ethyl acetate = 95/5) thus affording 0.060 g of the title compound (31% yield).
¹H-NMR (200 MHz; CDCl₃) δ (ppm): 1.44-1.65 (m, 2 H, *CH₂); 2.29-*2.40 (m, H, *CH);* 2.89-2.96 (m, H, *CH);* 3.71 (s, 3 H, OCH₃), 6.97-6.99 (d, H, J = 4 Hz, *CH),* 7.61-7.63 (d, H, J = 4 Hz, *CH).*

### Example 20

### (1S, 2S)-trans-[2-(2-chloro-5-thenoyl)]-cyclopropane-1-carboxylic acid

To a solution of the compound of example 19 (0.055 g, 0.22 mmol) in dioxane (1 ml) a solution of potassium hydroxide (0.017 g, 0.30 mmol) in water (1 ml) was added. The reaction mixture was stirred at room temperature for 4 h. Then, 2 ml of water were added, the two phases were separated and the aqueous layer was extracted once with diethyl ether. The aqueous layer was acidified with 10% HCl, then extracted three times with diethyl ether. The combined organic extracts were washed with a saturated solution of sodium chloride and dried over anhydrous sodium sulfate. The solvent was eliminated with a rotary evaporator. The crude product was minced with n-hexane, filtered under reduced pressure and dried with a high vacuum pump. 0.051 g of pure title compound was obtained (98% yield).
¹H-NMR (200 MHz; CDCl₃) δ (ppm): 1.55-1.72 (m, 2 H); 2.32-2.41 (m, H); 2.93-3.02 (m, H); 6.98-7.00 (d, H, J = 4 Hz), 7.63-7.65 (d, H, J = 4 Hz).
¹³C-NMR (200 MHz; CDCl₃) δ (ppm): 17.86, 23.27, 26.12, 123.00, 130.78, 142.86, 152.08, 179.59, 201.31.
e.e. (HPLC λ, = 254 nm) >99%

## Claims

1. Compounds of formula (**I**) 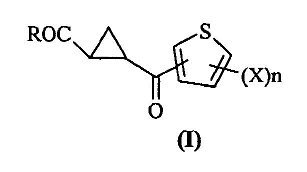 wherein
R is hydroxy, linear or branched C₁-C₆ alkoxy, phenoxy, benzyloxy, a group -N(R¹R²) wherein R¹ is hydrogen, linear or branched C₁-C₄ alkyl, benzyl, phenyl and R² is hydrogen or linear or branched C₁-C₄ alkyl, or R is a glycoside residue or a primary alkoxy residue from ascorbic acid, optionally having one or more hydroxy groups alkylated or acylated by linear or branched C₁-C₄ alkyl or acyl groups;
X is a halogen atom selected from the group consisting of fluorine, chorine or bromine, preferably chlorine;
n is an integer of 1 or 2
and pharmaceutically acceptable salts thereof.

2. Compounds of formula (**I**) wherein the halogen atom is chlorine.

3. Compounds according to claims 1 - 2 wherein n is 1.

4. Compounds according to any one of claims 1 - 3 wherein R is hydroxy.

5. Compounds according to any one of claims 1 - 3 wherein R is methoxy.

6. Compounds according to any one of claims 1 - 3 wherein R is ethoxy.

7. Compounds according to any one of claims 1 - 3 wherein R is a glycoside residue selected from an optionally alkylated or acylated beta D-glucopyranosyloxy or 6-deoxygalactopyranosyloxy residue.

8. Compounds according to claim 7 wherein R is a galactopyranosyl residue.

9. Compounds according to any one of claims 1 - 3 wherein R is a primary alkoxy residue from ascorbic acid.

10. A compound selected from:
2-(2-chloro-4-thenoyl)-cyclopropane-1-carboxylic acid,
methyl-2-(2-chloro-4-thenoyl)-cyclopropane-1 -carboxylate,
ethyl-2-(2-chloro-4-thenoyl)-cyclopropane-1-carboxylate,
2-(2-chloro-5-thenoyl)-cyclopropane-1-carboxylic acid,
methyl-2-(2-chloro-5-thenoyl)-cyclopropane-1-carboxylate,
ethyl-2-(2-chloro-5-thenoyl)-cyclopropane-1-carboxylate,
2-(2,3-dichloro-4-thenoyl)-cyclopropane-1-carboxylic acid,
methyl-2-(2,3-dichloro-4-thenoyl)-cyclopropane-1-carboxylate,
ethyl-2-(2,3-dichloro-4-thenoyl)-cyclopropane-1-carboxylate.

11. Pharmaceutical compositions comprising a compound of any one of claims 1 - 10.

12. The use of a compound any one of claims 1 - 10 for the preparation of medicaments for the treatment of any one of the following conditions:
neurodegenerative disorders including Parkinson's syndrome, Huntington's chorea, Senile Dementia Alzheimer's type, Amiotrophic Lateral Sclerosis;
inflammatory disorders of the central and/or peripheral nervous system including multiple sclerosis, Guillain Barre Syndrome and other neurophaties;
infectious diseases caused by viruses, bacteria and other parasites including malaria and septic shock;
immunitary disorders and therapeutic treatments aimed at modifying biological responses;
neoplastic disorders including lymphomas and malignant blood disorders;
convulsive Disorders, including variants of *Grand mal* and *petit mal* epilepsy and Partial Complex epilepsy;
ischemic disorders including stroke;
cardiac arrest or insufficiency and hemorrhagic shock, carbon monoxide poisoning, near drawning;
traumatic damage to the brain and spinal cord;
tremor syndromes and different movement disorders;
psychiatric disorders including anxiety, insomnia, depression and schizophrenia;
nicotine addiction and other addictive disorders including alcoholism and cannabis, benzodiazepine, barbiturate, morphine and cocaine dependence;
cataract formation and aging of the eye.

## Patentansprüche

1. Verbindungen der Formel (I) 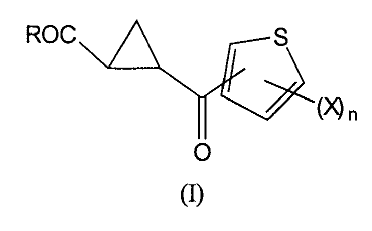 worin
R für Hydroxy, lineares oder verzweigtes C₁-C₆-Alkoxy, Phenoxy, Benzyloxy, eine N(R¹R²)-Gruppe, worin R¹ Wasserstoff, lineares oder verzweigtes C₁-C₄-Alkyl, Benzyl, Phenyl und R² Wasserstoff oder lineares oder verzweigtes C₁-C₄-Alkyl darstellt, oder R für einen Glycosidrest oder einen primären Alkoxyrest der Ascorbinsäure gegebenenfalls mit jeweils einer oder mehreren durch lineare oder verzweigte C₁-C₄-Alkyl- oder Acylgruppen alkylierten bzw. acylierten Hydroxygruppen steht;
X für ein Halogenatom ausgewählt aus der Gruppe bestehend aus Fluor, Chlor, Brom, bevorzugt Chlor, steht;
n für eine ganze Zahl 1 oder 2 steht;
und deren pharmazeutisch verträgliche Salze.

2. Verbindungen der Formel (I), worin das Halogenatom Chlor ist.

3. Verbindungen gemäß Anspruch 1 bis 2, worin n 1 ist.

4. Verbindungen gemäß einem der Ansprüche 1 bis 3, worin R für Hydroxy steht.

5. Verbindungen gemäß einem der Ansprüche 1 bis 3, worin R für Methoxy steht.

6. Verbindungen gemäß einem der Ansprüche 1 bis 3, worin R für Ethoxy steht.

7. Verbindungen gemäß einem der Ansprüche 1 bis 3, worin R für einen Glycosidrest ausgewählt aus der Gruppe bestehend aus einem gegebenenfalls alkylierten oder acylierten beta-D-Glucopyranosyloxy- oder 6-Deoxygalactopyranosyloxyrest steht.

8. Verbindungen gemäß Anspruch 7, worin R für einen Galactopyranosylrest steht.

9. Verbindungen gemäß einem der Ansprüche 1 bis 3, worin R für einen primären Alkoxyrest der Ascorbinsäure steht.

10. Eine Verbindung ausgewählt aus:
2-(2-Chlor-4-thenoyl)-cyclopropan-1-carbonsäure,
Methyl-2-(2-chlor-4-thenoyl)-cyclopropan-1-carboxylat,
Ethyl-2-(2-chlor-4-thenoyl)-cyclopropan-1-carboxylat,
2-(2-Chlor-5-thenoyl)-cyclopropan-1-carbonsäure,
Methyl-2-(2-chlor-5-thenoyl)-cyclopropan-1-carboxylat,
Ethyl-2-(2-chlor-5-thenoyl)-cyclopropan-1-carboxylat,
2-(2,3-Dichlor-4-thenoyl)-cyclopropan-1-carbonsäure,
Methyl-2-(2,3-dichlor-4-thenoyl)-cyclopropan-1-carboxylat, und
Ethyl-2-(2,3-dichlor-4-thenoyl)-cyclopropan-1-carboxylat.

11. Pharmazeutische Zusammensetzungen enthaltend eine Verbindung gemäß einem der Ansprüche 1 bis 10.

12. Verwendung einer Verbindung gemäß einem der Ansprüche 1 bis 10 zur Herstellung von Arzneimitteln für die Behandlung einer der folgenden Krankheiten: Neurodegenerative Störungen einschließlich das Parkinson-Syndrom, Chorea Huntington , senile Demenz vom Alzheimer-Typ, Amyotrophische Lateralsklerose; Entzündungskrankheiten des Zentralen und/oder Peripheren Nervensystems einschließlich Multiple Sklerose, Guillain Barre Syndrom und andere Neuropathien;
von Viren, Bakterien und anderen Parasiten hervorgerufene Infektionskrankheiten einschließlich Malaria und septischer Schock; Immunkrankheiten und Therapiebehandlungen, die auf die Abänderung der biologischen Antworten abzielen;
neoplastische Störungen einschließlich Lymphome und maligne Blutkrankheiten;
konvulsive Störungen, einschließlich Varianten der Grand-mal- und Petit-mal-Epilepsie und komplex-fokale Epilepsieanfälle;
ischämische Störungen einschließlich Schlaganfall;
Herzstillstand oder -insuffizienz und hämorrhagischer Schock, Kohlenmonoxidvergiftung, beinahes Ertrinken;
traumatische Verletzungen des Gehirns und der Wirbelsäule;
Tremorsyndrome und verschiedene Bewegungsstörungen;
psychiatrische Störungen einschließlich Angstzustände, Schlaflosigkeit, Depressionen und Schizophrenie;
Nikotinabhängigkeit und andere Abhängigkeitsstörungen einschließlich Alkoholismus und Cannabis-, Benzdiazepin-, Barbiturat-, Morphin- und Cocainabhängigkeit. Ausbildung von Katarakten und Alterung des Auges.

## Revendications

1. Composés de formule (I) 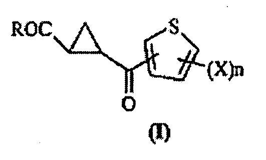 dans laquelle
R représente un groupe hydroxyle, alcoxy en C₁-C₆ linéaire ou ramifié, phénoxy, benzyloxy, -N(R¹R²) dans lequel R¹ représente un hydrogène, un groupe alkyle en C₁-C₄ linéaire ou ramifié, benzyle ou phényle et R² représente un hydrogène ou un groupe alkyle en C₁-C₄ linéaire ou ramifié, ou R représente un résidu glycoside ou un résidu alcoxy primaire dérivé d'acide ascorbique, portant éventuellement un ou plusieurs groupes hydroxyle alkylés ou acylés par des groupes alkyle ou acyle en C₁-C₄ linéaires ou ramifiés ;
X représente un atome d'halogène choisi dans le groupe constitué par le fluor, le chlore et le brome, de préférence le chlore ;
n est un entier égal à 1 ou 2,
et les sels pharmaceutiquement acceptables de ceux-ci.

2. Composés de formule (I) dans laquelle l'atome d'halogène est un chlore.

3. Composés selon les revendications 1 et 2 dans lesquels n vaut 1.

4. Composés selon l'une quelconque des revendications 1 à 3 dans lesquels R représente un groupe hydroxyle.

5. Composés selon l'une quelconque des revendications 1 à 3 dans lesquels R représente un groupe méthoxy.

6. Composés selon l'une quelconque des revendications 1 à 3, dans lesquels R représente un groupe éthoxy.

7. Composés selon l'une quelconque des revendications 1 à 3 dans lesquels R représente un résidu glycoside choisi parmi un résidu beta-D-glucopyranosyloxy ou 6-déoxy-galactopyranosyloxy éventuellement alkylé ou acylé.

8. Composés selon la revendication 7 dans lesquels R représente un résidu galactopyranosyle.

9. Composés selon l'une quelconque des revendications 1 à 3 dans lesquels R représente un résidu alcoxy primaire dérivé d'acide ascorbique.

10. Composé choisi parmi :
l'acide 2-(2-chloro-4-thénoyl)-cyclopropane-1-carboxylique,
le 2-(2-chloro-4-thénoyl)-cyclopropane-1-carboxylate de méthyle,
le 2-(2-chloro-4-thénoyl)-cyclopropane-1-carboxylate d'éthyle,
l'acide 2-(2-chloro-5-thénoyl)-cyclopropane-1-carboxylique,
le 2-(2-chloro-5-thénoyl)-cyclopropane-1-carboxylate de méthyle,
le 2-(2-chloro-5-thénoyl)-cyclopropane-1-carboxylate d'éthyle,
l'acide 2-(2,3-dichloro-4-thénoyl)-cyclopropane-1-carboxylique,
le 2-(2,3-dichloro-4-thénoyl)-cyclopropane-1-carboxylate de méthyle,
le 2-(2,3-dichloro-4-thénoyl)-cyclopropane-1-carboxylate d'éthyle.

11. Compositions pharmaceutiques comprenant un composé selon l'une quelconque des revendications 1 à 10.

12. Utilisation d'un composé selon l'une quelconque des revendications 1 à 10 pour la préparation de médicaments destinés au traitement de l'un quelconque des états suivants :
troubles neurodégénératives incluant le syndrome parkinsonien, la chorée de Huntington, la démence sénile de type Alzheimer, la sclérose latérale amyotrophique ;
troubles inflammatoires du système nerveux central et/ou périphérique incluant la sclérose en plaques, le syndrome de Guillain-Barré et d'autres neuropathies ;
maladies infectieuses causées par des virus, des bactéries et d'autres parasites incluant le paludisme et le choc septique ;
troubles immunitaires et traitements thérapeutiques visant à modifier des réponses biologiques ;
troubles néoplasiques incluant les lymphomes et les troubles sanguins malins ;
troubles convulsifs incluant les variantes grand mal et petit mal d'épilepsie et l'épilepsie partielle complexe ;
troubles ischémiques incluant l'accident vasculaire cérébral ;
arrêt ou insuffisance cardiaque et choc hémorragique, empoisonnement au monoxyde de carbone, quasi-noyade ;
lésion traumatique du cerveau et de la moelle épinière ;
syndromes de tremblements et différents troubles du mouvement ;
troubles psychiatriques incluant l'anxiété, l'insomnie, la dépression et la schizophrénie ;
dépendance à la nicotine et autres troubles addictifs incluant l'alcoolisme et la dépendance au cannabis, à la benzodiazépine, au barbiturate, à la morphine et à la cocaïne ;
formation d'une cataracte et vieillissement de l'oeil.
